# EUROPEAN PATENT APPLICATION

(11) **EP 3 298 908 A2**
(43) Date of publication of application: **28.03.2018**
(21) Application number: 17176658.7
(22) Date of filing: 19.06.2017
(51) Int. Cl.: A23L 33/175, A23L 33/18, A61K 38/01, A61K 38/19

(54) **PROTEIN-BASED AMINO ACID COMPOSITION FOR USE PRIOR TO EXERCISE**

(30) Priority: 29.06.2016 PL 41776916
(71) Applicant: Olimp Laboratories Sp. Z O.O., 39-200 Debica (PL)
(72) Inventor: Kaczka, Piotr, 35-106 Kielanówka (PL); Kubicka, Katarzyna, 58-302 Walbrzych (PL); Batra, Amit, 54-206 Wroclaw (PL)
(74) Representative: Kondrat, Mariusz

(57) **Abstract**

The subject of the invention is an amino acid composition obtained by combining any hydrolysates of protein sources, any hydrolysates of protein sources and free amino acids, or only free amino acids, for use prior to exercise, characterised in that, it has an amino acid profile, wherein the mutual weight ratio of the respective amino acids is identical to that found in the Intracellular liquid of human muscle cells in the state of homeostasis.

## Description

The subject of the invention is an amino acid composition obtained by combining any hydrolysates of protein sources, any hydrolysates of protein sources and free amino acids, or only free amino acids, for use prior to exercise.
Prior art amino acid products are exclusively based on protein hydrolysates. Therefore, their amino acid composition corresponds to the composition of the direct source of raw material, which are mostly or exclusively whey or beef protein.
Prior art products containing the complex of amino acids, are not based on the quantitative concept of the relative ratio of individual amino acids, but only provide the total sum of the amino acids produced by the manufacturer in the daily portion. Mutual amino acid ratios are characteristic for the source (whey protein, collagen, chicken meat, beef) and are not compiled for the purpose of using the product. Every excess and insufficiency of amino acids is detrimental to the body, especially the physically active, because it disturbs the balance of biochemical processes in the muscle cells (in other body cells too), may reduce the effectiveness of metabolism and the whole effort.

The aim of the invention was to create a product that would contain amino acids of animal origin, however, with an appropriate amino acid profile, beneficial to the human body during exercise (workout).
Given that, human muscle tissue has its own characteristic amino acid profile, a product has been created that will maximize its amino acid composition. The content of amino acids both qualitatively and quantitatively in human skeletal muscle contractions is presented on the following web pages:
http://www.uniprot.org/uniprot/Q7Z7J6
http://www.uniprot.org/uniprot/P12882
http://www.uniprot.org/uniprot/P05976

The composition according to the invention is a product intended for adults who practice sports, for use before training. The composition of the tablet was inspired by the amino acid profile of the intracellular fluid (cytoplasm) of the skeletal muscle cells. Amino acids are therefore delivered from the tablet exactly in the proportions needed for effective reconditioning of contractile cells.
This composition is almost completely devoid of fat.
The protein that forms the base of the composition is enzymatically hydrolysated, so that the human body can absorb it very quickly. In addition, the composition contains free amino acids whose rate of assimilation is also fast and the amino acid profile of the invention itself is unique and makes it as close as possible to the relationship between amino acids in the cytoplasm of the muscle cell.

The subject of the invention was to create an amino acid composition based on an innovative concept of the quantitative relationship between the various amino acids which is identical with the amino acid profile typical of homeostasis fluid in the intracellular (cytoplasmic) muscle cell. This concept is far different in its assumptions compared to the amino acid products previously found in the quality approach characterized by the use of protein hydrolysate (whey, collagen, chicken meat, beef, fish), with source specific, rather than human muscle proteins, amino acids.
The subject of the invention is an amino acid composition obtained by combining any hydrolysates of protein sources, any hydrolysates of protein sources and free amino acids or only free amino acids, for use prior to exercise, characterized in that it has an amino acid profile, in which the weight ratio of individual amino acids is identical with the weight ratio of the intracellular fluid of human muscle cells in the state of homeostasis.
Preferably, the composition further comprises L-glutamine, Taurine, L-alanine, L-lysine HCl, L-valine, L-isoleucine, L-threonine, L-methionine, L-ornithine L-aspartate, wherein the amino acids have a mutual weight ratio corresponding to the mutual weight ratio of the amino acids present in the human intracellular fluid in a state of homeostasis.
Preferably, the composition contains amino acids in the following weight portions: L-glutamine from 498 to 747, L-alanine from 101 to 152, L-proline from 36 to 55, Glycine from 35 to 53, L-lysine from 21 to 32, L-valine (BCAA) from 15 to 23, L-arginine from 12 to 18, L-threonine from 9 to 14, L-isoleucine (BCAA) from 8 to 13, L-asparagine from 8 to 13, L-methionine from 7 to 11, L-serine from 5 to 8, L-leucine (BCAA) from 4.6 to 6.8, L-phenylalanine from 3 to 4.6, L-tyrosine from 1.4 to 2, L-histidine from 1.1 to 1.7, L-tryptophan from 0.18 to 0.22, L-cysteine from 0.08 to 0.12, Taurine from 50 to 75, L-ornithine from 12 to 18.
Preferably, the composition further comprises the substances necessary to prepare the pharmaceutical formulation or dietary supplement.
Preferably, the composition is in the form of a powder, granule, tablet or effervescent tablet.

The composition according to the invention is an amino acid product for use prior to training, the composition is designed for the optimal ratio of the amino acids during exercise. The protein contributes to the growth and maintenance of muscle mass.
This product is intended for physically active adults.
Recommended daily dose: the formulation is to be used before workout, depending on body weight:
up to 80 kg - 5 tablets.
over 80 kg - 8 tablets.
Do not exceed recommended daily serving. The composition according to the invention can not be used as a substitute for a differentiated diet.

The composition of the invention consists of a filler material - microcrystalline cellulose; L-glutamine, bovine protein hydrolyzate, L-alanine, taurine, anti-caking agent - magnesium salts of fatty acids, silicon dioxide; L-lysine hydrochloride, L-valine, L-ornithine L-aspartate, L-isoleucine, L-threonine, L-methionine.

Amino acids are derived from the bovine protein hydrolyzate, which was supplemented with individual, free amino acids and dipeptide (L-ornithine L-aspartate) in order to achieve a unique qualitative-quantitative composition identical with the amino acid profile of the intracellular fluid of myocytes (muscle cells) in the state of homeostasis (balance of the body).

The composition is intended for physically active adults to use before exercise to maintain the internal balance (homeostasis) of the muscle cell and to prolong the effectiveness of exercise.

The invention has been described in the embodiments.

### Example 1 - Description of the effectiveness

The quantitative proportions of individual amino acids were selected on the basis of the mutual ratio of amino acids in intracellular fluid in the state of homeostasis (balance of the body). The longer the cellular state of the balance (homeostasis) is maintained, the more effective, for a long period of time, physiological processes such as energy production or muscle protein synthesis (filamentous fibrils made of actin and myosin proteins) will occur.

In the composition according to the invention, the relative proportions in the amino acid complex have been selected with respect to the aim of maintaining the balance in the cell as the only way to effectively operate during exercise.

### Example 2

**Content of individual amino acids in the composition**

| **Reference amino acid profile*** | **[mg]** | **[mg]** | **[mg]** | **[mg]** | **[mg]** | **[mg]** |
|---|---|---|---|---|---|---|
| **Total amount of amino acids** | **831,3** | **1039** | **1246,9** | **5195** | **8312** | **53280** |
| L-glutamine | 498,6 | 623,2 | 747,8 | 3116,0 | 4985,6 | 31958,9 |
| L-alanine | 101,3 | 126,6 | 151,9 | 633,1 | 1013,0 | 6493,6 |
| L-proline | 36,4 | 45,5 | 54,6 | 227,5 | 364,0 | 2333,3 |
| Glycine | 35,3 | 44,1 | 52,9 | 220,5 | 352,8 | 2261,5 |
| L-lysine | 21,5 | 26,9 | 32,3 | 134,3 | 214,9 | 1377,4 |
| L-valine (BCAA) | 15,4 | 19,3 | 23,2 | 96,7 | 154,7 | 991,8 |
| L-arginine | 11,8 | 14,8 | 17,8 | 73,9 | 118,3 | 758,3 |
| L-threonine | 9,2 | 11,5 | 13,8 | 57,4 | 91,9 | 588,8 |
| L-isoleucine (BCAA) | 8,6 | 10,8 | 13 | 54,2 | 86,7 | 555,6 |
| L-asparagine | 8,5 | 10,6 | 12,7 | 52,9 | 84,6 | 542,1 |
| L-methionine | 7,3 | 9,1 | 10,9 | 45,3 | 72,5 | 464,8 |
| L-serine | 5 | 6,3 | 7,6 | 31,4 | 50,3 | 322,2 |
| L-leucine (BCAA) | 4,6 | 5,7 | 6,8 | 28,4 | 45,4 | 290,8 |
| L-phenylalanine | 3 | 3,8 | 4,6 | 18,9 | 30,2 | 193,8 |
| L-tyrosine | 1,4 | 1,7 | 2 | 8,7 | 14,0 | 89,5 |
| L-histidine | 1,1 | 1,4 | 1,7 | 6,8 | 10,8 | 69,5 |
| L-tryptophan | 0,16 | 0,2 | 0,24 | 1,1 | 1,8 | 11,7 |
| L-cysteine | 0,08 | 0,1 | 0,12 | 0,3 | 0,5 | 3,0 |
| Taurine | 50 | 62,5 | 75 | 312,5 | 500,0 | 3205,1 |
| L-ornithine | 2 | 15,0 | 18 | 75,0 | 120,0 | 769,2 |

| | | | | | | |
|---|---|---|---|---|---|---|
| * Deviations from the reference amino acid profile may occur. This is typical for products of natural origin. | | | | | | |

### Example 3 Tablet composition

The tablet was made by the use of bovine protein hydrolyzate and the addition of individual amino acids in order to obtain an adequate relative ratio of individual amino acids. In addition, the tablet may contain acceptable excipients such as filler / binder, glidant or coating agent.

The tablet can be made by wet granulation as well as in the direct compensation process.

| Components | Quantity [mg]: |
|---|---|
| bovine protein hydrolyzate | 188 |
| L-glutamine | 605 |
| Taurine | 62,5 |
| L-alanine | 110 |
| L-lysine HCl | 25 |
| L-valine | 15 |
| L-isoleucine | 8 |
| L- threonine | 8 |
| L-methionine | 7,5 |
| L-ornithine L-aspartate | 15 |
| Comprecell 102 | 870 |
| Aerosil 200 | 18 |
| Magnesium stearate | 18 |
| Total: 1950 mg | |

## Claims

1. Amino acid composition obtained by combining any hydrolysates of protein sources, any hydrolysates of protein sources and free amino acids, or only free amino acids, for use prior to exercise, **characterized in that,** it has an amino acid profile, wherein the mutual weight ratio of the respective amino acids is identical to that found in the Intracellular liquid of human muscle cells in the state of homeostasis.

2. Composition according to claim 1, **characterized in that,** it further comprises L-glutamine, Taurine, L-alanine, L-lysines HCl, L-valine, L-isoleucine, L-threonine, L-methionine, L-ornithine L-aspartate, wherein the amino acids have a mutual weight ratio corresponding to the mutual weight ratio of the amino acids present in the intracellular fluid of the human in a state of homeostasis.

3. Composition according to claim 1, **characterized in that,** it comprises amino acids in the following weight proportion:
- L-glutamine from 498 to 747,
- L-alanine from 101 to 152,
- L-proline from 36 to 55,
- Glycine from 35 to 53,
- L-lysine from 21 to 32,
- L-valine (BCAA) from 15 to 23,
- L-arginine from 12 to 18,
- L-threonine from 9 to 14,
- L-isoleucine (BCAA) from 8 to 13,
- L-asparagine from 8 to 13,
- L-methionine from 7 to 11,
- L-serine from 5 to 8,
- L-leucine (BCAA) from 4.6 to 6.8,
- L-phenylalanine from 3 to 4.6,
- L-tyrosine from 1.4 to 2,
- L-histidine from 1.1 to 1.7,
- L-tryptophan from 0.18 to 0.22,
- L-cysteine from 0.08 to 0.12,
- Taurine from 50 to 75,
- L-ornithine from 12 to 18.

4. Composition according to claim 1, **characterised in that,** it further comprises the substances necessary to prepare a pharmaceutical formulation or dietary supplement.

5. Composition according to claim 1, **characterised in that,** it is in the form of a powder, granule, tablet or effervescent tablet.
